# EUROPEAN PATENT APPLICATION

(11) **EP 2 614 831 A1**
(43) Date of publication of application: **17.07.2013**
(21) Application number: 13158131.6
(22) Date of filing: 27.08.2009
(51) Int. Cl.: A61K 38/17, A61K 38/22

(54) **Adiponectin for treating pulmonary disease**

(30) Priority: 27.08.2008 JP 2008217721
(62) Divisional of application: 09788056.1
(71) Applicant: OTSUKA PHARMACEUTICAL CO., LTD., Chiyoda-ku Tokyo 101-8535 (JP)
(72) Inventor: Kotosai, Kounori, Osaka, 541-0045 (JP); Kirima, Kazuyoshi, Osaka, 541-0045 (JP); Karasutani, Keiko, Osaka, 541-0045 (JP); Ohmoto, Yasukazu, Osaka, 541-0045 (JP); Yabuuchi, Yoichi, Tokushima 7710131 (JP)
(74) Representative: HOFFMANN EITLE

(57) **Abstract**

The present invention provides an agent for inhibiting alveolar airspace enlargement containing adiponectin or an agent for inhibiting alveolar wall destruction containing adiponectin. The pulmonary disease therapeutic agents of the present invention are highly safe drugs that possess an excellent effect of decreasing the deterioration of the pulmonary function, such as airflow limitation, and exhibit an extremely high effect of treating pulmonary disease accompanied by irreversible deterioration in pulmonary function, with fewer adverse side effects such as nausea, vomiting and gastric acid secretion.

## Description

### Technical Field

The present invention relates to an agent for treating pulmonary disease that inhibits abnormal alveolar airspace enlargement associated with destructive changes of alveolar .walls caused by chronic injury thereto and/or that inhibits destructive changes of alveolar walls.

### Background Art

Various known pulmonary diseases (PD) include acute bronchitis due to acute inflammation caused by viruses or bacteria; bronchial asthma mainly caused by the inflammatory response of the respiratory tract due to eosinophils; interstitial pneumonia caused by alveolar epithelial inflammation; lung cancer caused by cancer cells; and chronic obstructive pulmonary disease (COPD), which is known as a disease characterized by pulmonary chronic inflammation caused by various factors, especially smoking, leading to alveolar wall destruction and bronchial mucous gland hypertrophy resulting in shortness of breath, increased cough and sputum, etc. Particularly, a disease formerly called emphysema often complicates a disease formerly called chronic bronchitis to varying degrees. Today, the obstructive pulmonary disease relating to these two diseases is referred to as COPD.

It is said that COPD is characterized by the progressive development of airflow limitation (airway obstruction) (Non-patent Document 1).

Unlike chronic bronchitis in which respiratory tract hypersecretion and infection do not involve airflow limitation, irreversible airflow limitation is considered to result from peripheral airway lesions. Such pulmonary diseases include, in addition to the aforementioned chronic obstructive pulmonary disease including emphysema and chronic bronchitis, cystic fibrosis, bronchiectasis, tuberculosis, pneumoconiosis, and the like.

To date, with regard to drug therapy for pulmonary disease, including COPD accompanied by irreversible airflow limitation, drugs such as β2-stimulants, anticholinergic agents and the like having bronchodilatory activity are used for temporally preventing or suppressing the symptoms. However, these drugs having bronchodilatory activity cannot decrease, for a long period of time, the deterioration of the pulmonary function, which is the most important clinical indicator.

Many large-scale clinical studies have examined the effects of therapy using, as inhalants, steroids having a potent inhibitory effect on the production of cytokines; however, several documents have reported that steroids could not decrease the deterioration of the pulmonary function over a long period of time (see Non-patent Documents 2 to 4).

Regarding the effects of PD therapy using drugs having inhibitory effects on the production of active oxygen, no reliable clinical studies have been conducted.

According to a clinical study, N-acetylcystein, an antioxidant that is considered to have a mechanism of action similar to that of agents for inhibiting the production of active oxygen, can reduce the frequency of acute exacerbation of COPD (Non-patent Document 5). However, no documents have reported that N-acetylcystein exhibits an effect of decreasing the deterioration of the pulmonary function over a long period of time.

Further, for the treatment of PD, the use of drugs inhibiting phosphodiesterase IV activity has been discussed. However, it is reported that these drugs have adverse side effects such as nausea, vomiting and gastric acid secretion (Non-patent document 6).

As described above, no drugs that achieve satisfactory performance as a drug for treating chronic pulmonary disease, and that decrease the irreversible deterioration of the pulmonary function, i.e., that inhibit abnormal alveolar airspace enlargement associated with destructive changes of alveolar walls due to chronic injury thereto and/or that inhibit destructive changes of alveolar walls, have been developed.

### Citation List

### Patent Literature

- PTL 1:: WO1996139429
- PTL 2:: WO1999/21577
- PTL 3:: JapanesePatentNo.3018186
- PTL 4:: JapanesePatentNo.4147220
- PTL 5:: JapaneseUnexaminedPatentNo.2004-16074 Non Patent Literature
- NPL 1:: Pauwell, R.A.et.al, Am.J.Respir.Crit.CareMed., 2001(163),1256-1276
- NPL 2:: PauwelsRA, LodahlCG, LaitinenLA, SchoutenJP, PostmaDS, PrideNB, etal.N.Engl.J.Med., 1999(340), 1948-1953
- NPL 3:: VestboJ,SorensenT,LangeP,BrixA,TorreP, ViskumK, Lancet, 1999(353)1819-1823
- NPL 4:: BurgePS, CalverleyPM, JonesPW, SpencerS, AndersonJA, MaslenTK, BMJ,2000(320),1297-1303
- NPL 5:: C.Stey, J.Steurer, S.Bachmann, T.C.Medici, M.R.Tramer, Eur.Respir.J., 2000(16), 253-262
- NPL 6:: PeterJ.Barnes, N.EngI.J.Med., 2000(343)No.4, 269-280
- NPL 7:: Maeda, K.etal., Biocjem.Biophys.Res.Commun., 1996(221),286-289

### Summary of Invention

### Technical Problem

The object of the present invention is to provide a highly safe drug that is useful for treating a patient with pulmonary disease whose pulmonary function is irreversibly deteriorated.

### Solution to Problem

The present inventors prepared an animal model that displays pathogenic features clinically very similar to COPD accompanied by irreversible deterioration in pulmonary function, and conducted extensive research using this animal model. As a result, they found that adiponectin, which is known as a collagen-like secretory protein specifically secreted from adipose cells, inhibits pulmonary emphysema and pulmonary function disorders caused by elastase treatment, and arrived at a highly safe novel therapeutic agent that exhibits an extremely high effect of treating pulmonary disease accompanied by irreversible deterioration in pulmonary function, with fewer adverse side effects such as nausea, vomiting, and gastric acid secretion. The present invention was accomplished based on such findings.

The present invention provides an adiponectin-containing agent for inhibiting alveolar airspace enlargement, and an adiponectin-containing agent for inhibiting alveolar wall destruction.

Item 1. An agent for inhibiting alveolar airspace enlargement, comprising adiponectin.

Item 2. The agent according to Item 1, wherein the adiponectin is contained in an amount effective for inhibiting alveolar airspace enlargement associated with a destructive change of an alveolar wall.

Item 3. The agent according to Item 1 or 2, wherein the adiponectin is contained in an amount of 0.01 to 70 wt.%.

Item 4. The agent according to any one of Items 1 to 3, for use in the treatment of emphysema, chronic bronchitis, cystic fibrosis, bronchiectasis, tuberculosis, pneumoconiosis, or chronic obstructive pulmonary disease.

Item 5. An agent for inhibiting alveolar wall destruction, comprising adiponectin.

Item 6. The agent according to Item 5, wherein the adiponectin is contained in an amount effective for inhibiting a destructive change of an alveolar wall.

Item 7. The agent according to any one of Items 5 or 6, wherein the adiponectin is contained in an amount of 0.01 to 70 wt.%.

Item 8. The agent according to any one of Items 5 to 7, for use in the treatment of emphysema, chronic bronchitis, cystic fibrosis, bronchiectasis, tuberculosis, pneumoconiosis, or chronic obstructive pulmonary disease.

The agent for inhibiting alveolar airspace enlargement of the present invention contains adiponectin in an amount effective for inhibiting alveolar airspace enlargement associated with destructive changes of alveolar walls, and the agent for inhibiting alveolar wall destruction of the present invention contains adiponectin in an amount effective for inhibiting the alveolar wall destruction.

Adiponectin, which is an active ingredient of the agent for inhibiting alveolar airspace enlargement or the agent for inhibiting alveolar wall destruction of the present invention, is a collagen-like protein secreted from adipose cells.

Adiponectin, which is an active ingredient of the agent for inhibiting alveolar airspace enlargement or the agent for inhibiting alveolar wall destruction (hereinafter abbreviated as "pulmonary disease therapeutic agents") of the present invention, is an easily available and well-known substance. Commercially available adiponectin or adiponectin prepared by the known method shown in the reference example described later can be generally used in the invention.

Adiponectin genes of human and mouse are disclosed in Patent Document 1 as human Acrp30 and mouse Acrp30, and human adiponectin is disclosed as apM1 in Non-Patent Document 7 and Patent Document 2. The estimated amino acid sequence of the adiponectin polypeptide used in the pulmonary disease therapeutic agents of the present invention as an active ingredient is described in Patent Document 2; however, Patent Document 2 merely refers to the inhibitory effect of the polypeptide on smooth muscle cell growth, and its availability as an agent for preventing or improving arteriosclerosis, an agent for preventing or treating restenosis after angioplasty, and an anti-inflammatory agent.

Further, Patent Document 3 shows the usefulness of adiponectin, which suppresses, without macrophages or lymphocytes, clonal growth of relatively matured marrow monocytoid progenitor cells in the differentiation stage, as an anti-inflammatory agent and a propagation suppressor for monocytoid cell. Moreover, Patent Document 4 teaches that the C-terminal side spherical domain of adiponectin (corresponding to the amino acid sequences SEQ ID NOs: 114 to 239 of human adiponectin, and the amino acid sequences SEQ ID NOs: 111 to 242 of mouse adiponectin) has the effect of decreasing scavenger receptor A expression, and can be used as a preventive and therapeutic agent for arteriosclerosis. Similarly, Patent Document 5 refers to the availability of the C-terminal side spherical domain of adiponectin as an AMP-activated protein kinase activator.

The following adiponectin are commercially available from Bio Vendor (Czechoslovakia; http://www.biovendor.com/products/proteins): globular recombinant adiponectin (Adiponectin Globular Human (E. coli)), recombinant adiponectin produced from E. *coli* (Adiponectin Human E. *coli* His), recombinant adiponectin produced from human embryonic kidney cells (Adiponectin Human HEK293 Flag), trimeric recombinant adiponectin produced from human embryonic kidney cells (Adiponectin Human Trimeric form (HEK)), low- and high-molecular-weight adiponectin produced from human embryonic kidney cells (Adiponectin LMW and MMW oligomer-rich Human (HEK)), middle- and high-molecular-weight adiponectin produced from human embryonic kidney cells (Adiponectin MMW and HMW oligomer-rich Human (HEK)), etc.

A specific example of the polypeptide used in the pulmonary disease therapeutic agents of the present invention as an active ingredient is a polypeptide known as "adiponectin", encoded by the PCR product and shown in examples described later. The full-length DNA sequence of human adiponectin is 4, 527 bp in length, and the DNA sequence encoding an open reading frame (ORF) is 732 bp in length, as shown in SEQ ID NO: 2. The amino acid sequence encoded by the ORF is composed of 244 amino acid sequences, as shown in SEQ ID NO: 1, and the gene sequence has been registered as the GenBank accession number NM_004797. Further, the full-length DNA sequence of mouse adiponectin is 1, 276 bp in length, as shown in SEQ ID NO: 5, and the DNA sequence encoding ORF is 741 bp in length, as shown in SEQ ID NO: 4. The amino acid sequence encoded by the ORF is composed of 247 amino acid sequences, as shown in SEQ ID NO: 3, and the gene sequence has been registered as the GenBank accession number AF304466. Moreover, gene expression of human and mouse adiponectin specific to fat tissue has been recognized.

The amino acid sequence homology between human adiponectin and mouse adiponectin is 83.61%, and the DNA sequence homology of the ORF is 79.78%. Such a remarkably high level of homology implies that both have the same activity.

The present inventors found that adiponectin inhibits abnormal alveolar airspace enlargement associated with destructive changes of alveolar walls due to chronic injury, or exhibits pulmonary function improvement attained by inhibiting destructive changes of alveolar walls, and they concluded that adiponectin can be preferably used as a drug.

As described above, since adiponectin can inhibit abnormal alveolar airspace enlargement associated with destructive changes of alveolar walls due to chronic injury, it can be used for treating pulmonary diseases such as emphysema, chronic bronchitis, cystic fibrosis, bronchiectasis, tuberculosis, pneumoconiosis, and COPD.
Patent Document 1: WO1996/39429
Patent Document 2: WO1999/21577
Non-patent Document 7: Maeda, K. et al. , B.B.R.C., (1996) Vol. 221 No.2, pp286-289

The production of adiponectin for use as the active ingredient of the pulmonary disease therapeutic agents of the invention, preparation of the pulmonary disease therapeutic agents using adiponectin as an active ingredient, etc. are now described.

The designation of amino acids, peptides, nucleotide sequences, nucleic acids, etc. by abbreviations in this specification is in conformity with the rules of nomenclature recommended by IUPAC-IUB (IUPAC-IUB Communication on Biological Nomenclature, Eur. J. Biochem., 138, 9(1984)), "The Guidelines for Drafting of Specifications Etc. Containing Nucleotide Sequence or Amino Acid Sequence Information" (Edited by the Japanese Patent Office, (July, 1998) and the conventions in the relevant field of art.

Adiponectin can be provided in the form of a recombinant protein by the established genetic engineering techniques [e.g. Science, 224, 1431(1984): Biochem. Biophys. Res. Comm., 130, 692(1985); Proc. Natl. Acad. Sci., USA., 80, 5990 (1983)]. In this case, as the adiponectin (apM1) gene, the gene which was previously established by the present inventors can be used [Biochem. Biophys. Res. Commun., 221, 286-289 (1996)].

As an alternative, adiponectin can be produced by the conventional method for chemical synthesis in accordance with the information on the amino acid sequence encoded by said gene.

The production of adiponectin by a genetic engineering technique can be performed according to a method described in Reference Example 1 below. More specifically, the production comprises constructing a recombinant DNA with which the gene coding for the objective protein may be expressed in a host cell, introducing the DNA into the host cell to obtain a transformant and culturing the transformant.

In the specification, a polynucleotide (DNA molecule) encompasses not only double strand DNA but also single strand DNA including sense chains and antisense chains which compose them, and is not limited to a length thereof. Therefore, the polynucleotide encoding adiponectin includes the double strand DNA including genomic DNA and the single strand DNA (sense chain) including cDNA and the single strand DNA (antisense chain) having the sequence complementary to the sense chain and synthetic DNA fragments thereof unless otherwise mentioned.

The polynucleotide (DNA molecule) herein is not defined by a functional region, and can include at least one of an expression suppression region, a coding region, a leader sequence, an exon and an intron.

The polynucleotide also includes RNA and DNA. The polypeptide comprising the certain amino acid sequence and the polynucleotide comprising the certain DNA sequence include fragments, homologs, derivatives and mutants thereof.

The mutants of the polynucleotide (mutant DNA) include naturally occurring allelic mutants, not naturally occurring mutants, and mutants having deletion, substitution, addition and insertion. But, these mutants encode the polypeptide having substantially the same function as the function of the polypeptide encoded by the polynucleotide before the mutation.

The mutation of the polypeptide (modification of amino acid sequence) is not necessary to occur by the naturally occurring one, e.g., mutation or post-translational modification, and may be those artificially made by utilizing the naturally occurring protein (e.g., human adiponectin). The above mutants of the polypeptide include allelic variants, homologs and natural mutants having at least 80%, preferably 95% and more preferably 99% homology to the polypeptide before the mutation.

The homology of the polypeptide or the polynucleotide can be analyzed by measurement using FASTA program (Clustal, V., Methods MoI. Biol., 25, 307-318(1994)). As the most preferable and simple method for homology analysis, it is possible to exemplify the method in which the sequence is stored on a medium (e.g., flexible disc, CD-ROM, hard disc drive, external disc drive, DVD, etc.) capable of being read by computer and then known sequence database is searched in accordance with well-known searching procedure using the stored sequence. Specific examples of the known sequence database include the followings:
- DNA Database of Japan (DDBJ) (http: //www. ddbj . nig. ac. jp/) ; -Genebank (http://www. ncbi. nlm. nih.gov/web/Genebank/ Index.htlm); and
- the European Molecular Biology Laboratory Nucleic Acid Sequence Database (EMBL) (http://www.ebi.ac.uk/ebidocs/embl db.html).

Lots of searching algorithms for the homology analysis are available for those skilled in the art. One example thereof includes a program referred to as BLAST program. There are 5 BLAST procedures in this program. Three (BLASTN, BLASTX and TBLASTX) among them have been designed for checking the nucleotide sequence. Remaining two have been designed for checking the protein sequence (Coulson, Trends in Biotechnology, 12:76-80(1994); Birren, et al., Genome Analysis, 1:543-559(1997)).

In addition, additional programs, e.g., a sequence alignment program and a program for identifying the sequences more distantly separated are available in the art for analyzing the identified sequence.

The mutant DNA is silent (no change in an amino acid residue encoded by a mutated nucleic acid sequence) or conservative for the amino acid encoded by this. Examples of conservative amino acid substitution are shown below.

Original amino acid residue Conservative substituted amino acid residue

| | |
|---|---|
| Ala | Ser |
| Arg | Lys |
| Asn | Gln or His |
| Asp | Glu |
| Cys | Ser |
| Gln | Asn |
| Glu | Asp |
| Gly | Pro |
| His | Asn or Gln |
| Ile | Leu or Val |
| Leu | Ile or Val |
| Lys | Arg, Asn or Glu |
| Met | Leu or Ile |
| Phe | Met, Leu or Tyr |
| Ser | Thr |
| Thr | Ser |
| Trp | Tyr |
| Tyr | Trp or Phe |
| Val | Ile or Leu |

Generally, one or more codons encoding a Cys residue affects a disulfide bond of the particular polypeptide.

The substitution of the amino acid residue generally thought to affect characteristics of the protein includes the following:
a) the substitution of a hydrophobic residue with a hydrophilic residue, e.g., the substitution of Leu, Ile, Phe, Val or Ala with Ser or Thr;
b) the substitution of the amino acid residue other than Cys and Pro with Cys or Pro;
c) the substitution of the residue having an electrically positive side chain, e.g., Lys, Arg or His with an electrically negative residue, e.g., Glu or Asp; and
d) the substitution of the amino acid residue having an extremely large side chain, e.g., Phe with the amino acid residue having no side chain, e.g., Gly.

Adiponectin comprises the amino acid sequence of SEQ ID NO: 1 or 3 or the amino acid sequence having one or more amino acid deletions, insertions, substitutions or additions in the amino acid sequence of SEQ ID NO: 1 or 3, and has the alveolar airspace enlargement inhibitory activity and/or the pulmonary function improvement effect.

Adiponectin may be the polypeptide expressed in a protein expression system using *Escherichia coli* or a protein expression system using baculovirus (AcNPV) shown in Examples described later by gene recombination technology or the polypeptide obtained by chemically synthesizing.

As one specific example of the amino acid sequence of adiponectin, it is possible to exemplify one of SEQ ID NO:1 or 3. The amino acid sequence of adiponectin is not limited to one of SEQ ID NO: 1 or 3, and can be those (homologous ones) having a certain homology thereto. The homologous ones can include the polypeptides comprising the amino acid sequence having one or more amino acid deletions, insertions, substitutions or additions in the amino acid sequence of SEQ ID NO: 1 or 3 and having the alveolar airspace enlargement inhibitory activity and/or the alveolar wall destruction inhibitory activity, and the pulmonary function improvement effect (the activity of inhibiting abnormal alveolar airspace enlargement associated with destructive changes of alveolar walls due to chronic injury, and/or the activity of inhibiting destructive changes of alveolar walls).

Specifically, the pulmonary function improvement effects of adiponectin include an effect of improving or reducing a decrease in breathing capacity of lungs, or an increase in breathing resistance in a respiratory tract (especially the bronchoalveolar tract) of mammals such as human.

As a host cell, cells derived from eucaryotes and prokaryotes can be employed. The eucaryotic cell includes cells of vertebrates and cells of eucaryotic microorganisms. As the cell of a vertebrate, the monkey cell line COS (Cell, 23, 175 (1981)), the Chinese hamster ovarian cell line and the corresponding dihydrofolate reductase-deficient cell line (Proc. Natl. Acad. Sci., USA., 77, 4216 (1980)) and the like are often used but these are not exclusive choices.

As the expression vector of a vertebrate origin, a vector having a promoter sequence located upstream of the gene to be expressed, RNA (precursor) splice site, a polyadenylation site and a transcription terminating sequence can be generally used. If necessary, the vector may further have a replication origin. As an example of such expression vector, pSV2dhfr harboring an early promoter of SV40 can be mentioned (Mol. Cell. Biol., 1, 854 (1981)).

As the eucaryotic microorganisms, yeasts are generally used. Of these, yeasts of the genus Saccharomyces can be used with advantage. As the expression vector derived from a eucaryotic microorganism such as a yeast, pAM82 having a promoter for the acidphosphatase gene (Proc. Natl. Acad. Sci., USA., 80, 1 (1983)) can be utilized.

As the prokaryotic host, *Escherichia coli* and *Bacillus subtilis* are generally used. When they are used as hosts, using a plasmid vector capable of replicating in the host microorganism, it is possible to suitably use an expression plasmid obtained by incorporating a promoter and SD (Shain and Dalgarano) sequence and an initiation codon (e. g. , ATG) required for the initiation of protein synthesis at upstream of the gene so that the gene can be expressed in this vector. As said *Escherichia coli* as the host, E. *coli* K12 is generally used, and as the vector, pBR322 or its modification product is generally used. However, these are not exclusive choices but the various known bacterial strains and vectors can likewise be employed. Examples of the promoter that can be used are tryptophan (trp) promoter, lpp promoter, lac promoter, and PL/PR promoter.

As the vector for the insect cell, it is possible to exemplify a baculovirus vector (Takara) in which cDNA of adiponectin has been incorporated. Specifically, the expressed product of the present invention can be obtained by introducing the baculovirus expression vector in which cDNA of adiponectin has been incorporated into cultured cells BmN4 or larvae of silkworm *(Bombyx mori)* using nuclear polyhedrosis virus (BmNPV) of silkworm to express, and isolating from a culture medium or a silkworm body fluid by chromatography.

The expressed product of the present invention can also be obtained by incorporating the cDNA of adiponectin into the nuclear polyhedrosis virus (AcNPV) of *Autographa californica,* expressing it in Sf9 cells of *Spodoptera frugiperda* or Tn5 cells of *Trichoplusia ni,* and likewise purifying from a culture supernatant by chromatography.

Introduction of the resulting recombinant DNA into the host cell for transformation can be carried out according to the known method.

The resulting transformant can be cultured in accordance with the standard methods, whereby the objective recombinant protein is expressed and produced (accumulated or secreted) intracellularly, extracellularly or on the cell membrane. As a medium used for the culture, various media commonly used can be suitably selected and used according to the host cell employed. The culture can also be carried out under conditions suitable for the growth of the host cell.

If necessary, the adiponectin obtained in the above manner can be isolated and purified by various separation procedures utilizing the physical, chemical and other characteristics thereof (Biochemical Data Book II, 1175-1259, First Edition, 1st impression, Jun. 23, 1980, published by Tokyo Kagaku Dojin, K. K.; Biochemistry, 25 (25), 8274 (1986); Eur. J. Biochem., 163, 313 (1987), etc.). More specifically, said isolation and purification can be achieved by the conventional reconstitution treatment, treatment with a protein-precipitating (salting-out) agent, centrifugation, osmotic pressure shock method, sonication, ultrafiltration, various types of liquid chromatography such as molecular sieve chromatography (gel filtration), adsorption chromatography, ion-exchange chromatography, affinity chromatography, high-performance liquid chromatography (HPLC), etc., and dialysis as used either singly or in combination.

Alternatively, the adiponectin mentioned above can also be produced by the general method for chemical synthesis based on the amino acid sequence information. The method includes the conventional liquid-phase and solid-phase methods for peptide synthesis. In more detail, each of these methods includes the so-called stepwise elongation technique which comprises condensing component amino acids one after another for chain extension according to the amino acid sequence information, and the fragment condensation technique which comprises synthesizing fragment peptides each consisting of several amino acid residues in advance and coupling them together one after another according to said information.

A condensation method employed for the peptide synthesis can also be performed in accordance with the standard methods. Examples of the standard methods include an azide method, a mixed acid anhydrate method, a DCC method, an active ester method, an oxidation and reduction method, a DPPA (diphenylphosphorylazide) method, a DDC + additive (1-hydroxybenzotriazole, N-hydroxysuccinamide, N-hydroxy-5-norbornene-2,3-dicarboxyimide) method and Woodward method.

The solvent that can be used in those methods can be suitably selected from the solvents that are well known to be of use in peptide-forming condensation reactions. Examples thereof include N,N-dimethylformamide (DMF), dimethyl sulfoxide (DMSO), hexaphosphoramide, dioxane, tetrahydrofuran (THF), ethyl acetate, etc., and mixture thereof.

Upon the above peptide synthesis reaction, the amino acid not involved in the reaction or the carboxyl group in the peptide can be protected as lower alkyl ester such as methyl ester, ethyl ester and tert-butyl ester, and aralkyl ester such as benzyl ester, p-methoxybenzyl ester and p-nitrobenzyl ester generally by esterification.

The hydroxyl group in the amino acid such as tyrosine residue having a functional group in the side chain may be protected with acetyl, benzyl, benzyloxycarbonyl, tert-butyl or the like, although such protection is not necessarily indispensable.

Moreover, the guanidino group of an arginine residue can be protected with an appropriate protective group such as nitro, tosyl, p-methoxybenzenesulfonyl, methylene-2-sulfonyl, benzyloxycarbonyl, isobornyloxycarbonyl or adamantyloxycarbonyl.

The reactions for elimination of such protective groups from the protected amino acids or peptides, or from the final protein, can be carried out in accordance with the methods commonly used, such as a catalytic reduction method or method using liquid ammonia/sodium metal, hydrogen fluoride, hydrogen bromide, hydrogen chloride, trifluoroacetic acid, acetic acid, formic acid, or methanesulfonic acid, and the like.

The adiponectin thus obtained can be purified by the various methods such as methods using an ion-exchange resin, partition chromatography and gel chromatography, and a countercurrent distribution method commonly used in the field of peptide chemistry.

The pulmonary disease therapeutic agents of the present invention comprise adiponectin or a pharmacologically acceptable salt thereof as an active ingredient. The salt includes those with alkali metals, alkaline earth metals and ammonium, such as the sodium, potassium, lithium, calcium, magnesium, barium and ammonium salts. These salts can be produced by the methods well known in the art. The above-mentioned salt further includes acid addition salts which can be prepared by reacting adiponectin with a suitable organic or inorganic acid in the per se known manner. Examples of the acid addition salts are hydrochloride, hydrobromide, sulfate, bisulfate, acetate, oxalate, valerate, oleate, laurate, borate, benzoate, lactate, phosphate, p-toluenesulfonate (tosylate), citrate, maleate, fumarate, succinate, tartrate, sulfonate, glycolate, ascorbate, benzenesulfonate, napsylate and like salts.

The pulmonary disease therapeutic agents of the invention are generally provided and put to use in the form of a pharmaceutical preparation containing a pharmacologically effective amount of the active ingredient together with an appropriate pharmaceutical carrier.

The carrier that can be utilized in such pharmaceutical preparations includes various diluents and/or excipients, such as fillers, extenders, binders, humectants, disintegrants, surfactants, lubricants, and the like. These carriers are optionally used according to a unit dosage form of the resulting formulation.

The unit dosage forms of the pharmaceutical preparations can be selected from various forms according to the purpose of therapy. Typical examples include solid forms such as tablets, pills, powders, fine powders, granules and capsules, and liquid forms such as solutions, suspensions, emulsions, syrups and elixirs. These preparations are classified, by route of administration, into oral preparations, parenteral preparations, transnasal preparations, vaginal preparations, rectal suppositories, sublingual tablets, ointments, and the like, and each can be formulated and molded or otherwise processed by the established pharmaceutical procedure. Furthermore, such pharmaceutical preparations may contain various additives which can be formulated in ordinary pharmaceutical preparations, such as stabilizers, antibacterial agents, buffers, isotonizing agents, chelating agents, pH control agents and surfactants.

The stabilizer includes human serum albumin and those L-amino acids, saccharides and cellulose derivatives. These can be used each alone or in combination with a surfactant or the like. Particularly, such a combination may contribute to an enhanced stability of the active ingredient.

The L-amino acids are not particularly limited and include glycine, cysteine, glutamic acid, etc.

The saccharides are not particularly limited but include monosaccharides such as glucose, mannose, galactose and fructose; sugar alcohols such as mannitol, inositol and xylitol; disaccharides such as sucrose, maltose and lactose; and polysaccharides such as dextran, hydroxypropyl-starch, chondroitin sulfate and hyalluronic acid; and derivatives thereof.

The surfactants are not particularly limited and ionic and nonionic surfactants can be employed. Examples of the surfactants are polyoxyethylene glycol sorbitan alkyl esters, polyoxyethylene alkyl ethers, sorbitan monoacyl esters, and fatty acid glycerides.

The cellulose derivatives are not particularly limited but include methylcellulose, ethylcellulose, hydroxyethylcellulose, hydroxypropylcellulose, hydroxypropylmethylcellulose, carboxymethylcellulose sodium etc.

The saccharides can be used at least about 0.0001 mg, and preferably within the range of about 0.01 to about 10 mg per 1µg of the active ingredient. The surfactants can be used at least about 0.00001 mg, and preferably within the range of about 0.0001 to about 0.01 mg per 1 µg of the active ingredient. The human serum albumin can be used at least about 0.0001 mg, and preferably within the range of about 0.001 to about 0.1 mg per 1 µg of the active ingredient. The amino acids can be used within the range of about 0.001 to about 10 mg per 1µg of the active ingredient. The cellulose derivatives can be used at least about 0.00001 mg, and preferably within the range of about 0.001 to about 0.1 mg per 1 µg of the active ingredient. The amount of the active ingredient contained in the pharmaceutical preparations of the present invention can be suitably selected from a broad range. It is suitable that the amount of the active ingredient is typically about 0.00001 to about 70 wt.%, and preferably about 0.0001 to about 5 wt.%.

The buffer which may be optionally incorporated in the pharmaceutical preparations includes boric acid, phosphoric acid, acetic acid, citric acid, ε-aminocaproic acid, glutamic acid and the corresponding salts (e.g. salts with alkali metals or alkaline earth metals such as the sodium, potassium, calcium and magnesium salts). The isotonizing agent includes sodium chloride, potassium chloride, sugars and glycerine. The chelating agent includes sodium edetate and citric acid.

The pharmaceutical preparations of the invention can be prepared as a liquid preparation, and additionally can be made into lyophilized dosage form obtained by lyophilizing the pharmaceutical formulation, which is prepared at an appropriate concentration in use by dissolving in the buffer containing saline.

In molding the pharmaceutical composition of the invention into the tablet form, there can be used, as the carrier, various excipients such as lactose, sucrose, sodium chloride, glucose, urea, starch, calcium carbonate, kaolin, crystalline cellulose, silicic acid and potassium phosphate; binders such as water, ethanol, propanol, simple syrup, glucose solution, starch solution, gelatin solution, carboxymethyl cellulose, hydroxypropyl cellulose, methyl cellulose and polyvinyl pyrrolidone; disintegrators such as sodium carboxymethyl cellulose, calcium carboxymethyl cellulose, low-substituted hydroxypropyl cellulose, dry starch, sodium alginate, agar powder, laminaran powder, sodium hydrogen carbonate and calcium carbonate; surfactants such as polyoxyethylene sorbitan fatty acid ester, sodium lauryl sulfate and stearyl monoglyceride; disintegration inhibitors such as sucrose, stearin, cacao butter and hydrogenated oil; absorption promoters such as quaternary ammonium base and sodium lauryl sulfate; humectants such as glycerin and starch; adsorbents such as starch, lactose, kaolin, bentonite and colloidal silicic acid; and lubricants such as purified talc, stearic acid salt, boric acid powder and polyethylene glycol.

Furthermore, if necessary, the tablets obtained can be coated with the conventional coating materials to provide sugar-coated tablets, gelatin-coated tablets, enteric tablets, film-coated tablets or double- or multi-layer tablets.

The pills can be prepared using, as the carrier, excipients such as glucose, lactose, starch, cacao butter, hydrogenated vegetable oil, kaolin and talc; binders such as gum arabic powder, gum tragacanth powder, gelatin and ethanol, and disintegrators such as laminaran and agar.

In general, capsules can be prepared by mixing the active ingredient with the above-mentioned carriers according to a usual method to enclose the former in a hard gelatin capsule, soft gelatin capsule or the like.

The liquid preparation for oral administration includes solutions, emulsions, suspensions, syrup and elixirs. Each can be prepared using a conventional inert diluent, for example a pharmacologically acceptable vehicle inclusive of water. The liquid preparation may further be supplemented with various auxiliary agents such as a wetting agent, an emulsifier and/or a suspending agent and can be prepared by the established procedure.

The liquid preparation for parenteral administration, for example a sterile aqueous or nonaqueous solution, emulsion or suspension, can be prepared by using a diluent such as water, ethyl alcohol, propylene glycol, polyethylene glycol, ethoxylated isostearyl alcohol, polyoxylated isostearyl alcohol, polyethoxylated sorbitan fatty acid esters and vegetable oils such as olive oil. The liquid preparation may be supplemented with an organic ester which can be injected or infused, such as ethyl oleate. Such preparations may be further supplemented with the solubilizer, buffer, wetting agent, emulsifier, suspending agent, preservative, dispersant and other additives.

Such pharmaceutical preparations can be sterilized by filtration through a bacterial filter, incorporation of a bactericide, irradiation treatment, heat treatment, or the like. Moreover, such pharmaceutical preparations may each be provided in the form of a sterile solid composition, which can be dissolved in sterilized water or a suitable sterilizable medium for extemporaneous sterilization.

Rectal suppositories and vaginal preparations can be prepared by using carriers such as polyethylene glycol, cacao butter, a higher alcohol, a higher alcohol ester, gelatin and a semi-synthetic glyceride.

Ointments such as pastes, creams and gels can be prepared by using such a diluent or diluents as white petrolatum, paraffin, glycerin, cellulose derivatives, propylene glycol, polyethylene glycol, silicones, bentonite, and vegetable oils such as olive oil.

Pharmaceutical preparations for transnasal or sublingual administration can be prepared using the well-known standard excipient or excipients in the conventional manner.

Furthermore, if necessary, the pharmaceutical preparations of the invention can be supplemented with coloring agents, preservatives, perfumes, flavors, sweeteners or other pharmaceutical compositions.

The method for administrating the pharmaceutical preparation is not limited, but is selected according to the dosage form, the patient age, sex and other conditions, and the status of the disease. For example, the tablets, pills, solutions, suspensions, emulsions, granules and capsules are orally administered. The injections are intravenously administered either alone or in mixture with an ordinary infusion such as glucose and amino acid, and if necessary, administered alone intramuscularly, intradermally, subcutaneously or intraperitoneally. The suppositories are administered intrarectally. The vaginal preparations are administered in vagina, the nasal preparations are administered in nose, the sublingual preparations are administered in oral cavity, and the ointments are percutaneously administered topically.

To make the active ingredient of the present invention into a powder form, the ingredient may be pulverized according to usual methods. For example, the active ingredient may be pulverized using lactose, starch or the like, and then stirred to obtain a homogeneous mixture.

Of the administration methods, pulmonary administration (inhalation) is more advantageous than oral administration because lung alveoli are the target organs of the treatment, and direct exposure to the damaged alveolar tissue site is possible, which prevents generalized side effects.

The amount of adiponectin contained in the therapeutic agent of the present invention is not limited, and can be suitably selected from a wide range. Adiponectin is generally contained in an amount of about 0.01 to about 70 wt. %, preferably about 0 .1 to about 50 wt. %, and more preferably about 0.3 to about 30 wt.% in the pharmaceutical composition.

The amount of the active ingredient to be contained in the pharmaceutical preparation and the dosage are not particularly limited but can be selected from a broad range according to the desired therapeutic effect, method of administration, treatment period, and patient's age, sex and other conditions. The dosage is usually selected so that the blood concentration of the active ingredient will be preferably about 0.1 to about 500 µg/ml, and more preferably about 1 to about 50 µg/ml. This preparation can be administered once or in a few divided doses a day.

The dosage of the therapeutic agent of the invention is suitably selected according to the method of use, the patient's age, sex and other conditions, and the severity of the disease. The amount of adiponectin may be usually about 0.1 µg to about 20 mg.

### Advantageous Effects of Invention

The adiponectin-containing agent for inhibiting alveolar airspace enlargement or the adiponectin-containing agent for inhibiting alveolar wall destruction of the present invention possesses an excellent effect of decreasing the deterioration of pulmonary function, such as airflow obstruction, and is highly effective for the treatment of pulmonary disease accompanied by irreversible deterioration in pulmonary function.

The adiponectin-containing agent for inhibiting alveolar airspace enlargement or the adiponectin-containing agent for inhibiting alveolar wall destruction of the present invention is a highly safe drug with fewer adverse side effects such as nausea, vomiting, gastric acid secretion, etc.

### Brief Description of Drawings

Fig. 1 is a graph showing that adiponectin has a dose-dependent inhibitory activity on alveolar airspace enlargement caused by progressing destructive changes of alveolar walls.
Fig. 2 shows tissue sections indicating progressing destructive changes of alveolar walls, and the inhibitory activity of adiponectin on progressive alveolar wall destruction.

### Description of Embodiments

### Preparation Example 1

The pharmaceutical composition of the present invention in the injectable formulation was prepared by adding and mixing 100 µg/mL of human adiponectin (having the amino acid sequence of SEQ ID NO: 1), 0.01 mg/mL of Tween 80 (Polyoxyethylene (20) Sorbitan Monooleate; Polysorbate 80), 15 mg/mL of dextran 40, 0.1 mglmL of cysteine, and 1.0 mg/mL of HSA (human serum albumin) in 0.01 M citric acid-sodium citrate buffer (pH 6.0), filtrating the mixture (using a membrane filter of 0.22 µm), then sterilely dispensing the filtrate by 1 mL in a vial, and lyophilizing it. The formulation can be used by dissolving in 1 mL of saline in use.

### Preparation Example 2

The pharmaceutical composition of the present invention in the injectable formulation was prepared by adding 10 µg/0.1 mL of human adiponectin (having the amino acid sequence of SEQ ID NO: 1), 5 mg of cysteic acid, and 1 mg of human serum albumin (HSA) per vial in distilled water for the injection, filling the resulting solution in one vial by 1 mL and lyophilizing it.

### Preparation Example 3

The pharmaceutical composition of the present invention in the injectable formulation was prepared by adding 0.5 mg of adiponectin, 80 mg of sodium chloride, 20 mg of mannitol, 34.5 mg of sodium phosphate, and 45 mg of PEG in 10 ml of distilled water for the injection, followed by aseptic filtration, dispensing the filtrate by 1 mL in an aseptic vial, and lyophilizing it.

### Reference Example 1

### Production of a Recombinant Human Adiponectin

### (1) Expression of human adiponectin in Escherichia coli

### 1) human adiponectin PCR

The human adiponectin gene and the amino acid sequence encoded thereby have been deposited with the GenBank under the accession number of NM_004797 and D45371, respectively. The coding region (CDS) is shown as the sequence from 27th to 761st of the nucleotide sequence. Its deduced amino acid sequence is shown in SEQ ID NO:1. In this sequence, the 1st to 14th residues constitute a signal peptide and the 15th to 244th residues represent the mature human adiponectin.

The human adiponectin gene was amplified by PCR using the plasmid donated by Dr. Funahashi, the Second Department of Internal medicine, Osaka University School of Medicine as a template.

Designing so that the 693bp sequence from 69th to 761st of the nucleotide sequence of the human adiponectin would be amplified with an NdeI site at the 5'-end and a BamHI site at the 3'-end, PCR primers were produced using an automatic DNA synthesizer. The PCR primer sequences are shown in SEQ ID NO:6 (forward) and NO:7 (reverse).

### 2) Subcloning of the Human Adiponectin (apM1) Gene

The PCR product obtained in the above step 1) was subcloned into pT7 Blue T-Vector (Novagen) and it was confirmed that there was no mutation in its nucleotide sequence (pT7-apM1).

### 3) Construction of an Expression Vector

The expression vector pET3c (Novagen) was digested with NdeI and BamHI to recover a fragment of approximately 4600 bp. On the other hand, the pT7-apM1 obtained in the above step 1) was digested with NdeI and BamHI to recover a fragment of approximately 700 bp. These fragments were ligated and the expression vector thus obtained was named pET3c-apM1

### 4) Expression in Escherichia coli

The host *E*. *coli* strain BL21(DE3)pLysS was transformed with the pET3c-apM1 constructed in the above step 3) and cultured in 2xT.Y.Amp. (tryptone 16 g, yeast extract 10 g, and NaCl 5 g). When the organism had entered into the logarithmic growth phase, IPTG (isopropyl β-D-thiogalactopyranoside) was added for inducing the production of a recombinant human adiponectin (apM1). The E. *coli* cells before and after this IPTG induction and the inclusion body (the insoluble fraction of *E*. *coli*) after said IPTG induction were sampled and subjected to SDS-PAGE and Western blotting to confirm the expression of human adiponectin.

### 5) Results and Discussion

The expression product in E. *coli,* obtained in the above manner, was a 230-residue protein corresponding to the ¹⁵Gly to ²⁴⁴Asn, exclusive of the signal sequence, of the amino acid sequence of human adiponectin, with the addition of Met derived from the initiation codon at the N-terminus.

The E. *coli* obtained by the above procedure was analyzed by SDS-PAGE. As a result, an approximately 30 kD band could be confirmed in the *E*. *coli* cell and inclusion body after IPTG induction.

Then, Western blotting was performed using two kinds of antibodies (polyclonal antibodies (synthetic peptides)). Both antibodies reacted with said approximately 30 kD band, whereas no reaction was detected at all with the host *E*. *coli.*

The above approximately 30 kD band was excised to investigate the sequence of its 10 amino acid residues at N-terminal. The sequence was the same as the expected sequence, with the deletion of the N-terminal Met having been found in a minor population.

It became clear from the above results that the recombinant human adiponectin had been expressed as an approximately 30 kD protein. Most of the recombinant human adiponectin expressed had been intracellularly accumulated as an inclusion body.

### (2) Purification of the Recombinant human adiponectin (apM1) from E. coli

Purification of the recombinant human adiponectin from *E*. *coli* was carried out by the following 5-step procedure.

### 1) Culture of E. coli

The *E*. *coli* BL21 (DE3) pLysS (Novagen) transformed with the expression vector pET3c-apM1 was precultured in 2xT.Y.Amp.Cm. (tryptone 16 g, yeast extract 10 g, chloramphenicol 25 µg/ml, and NaCl 5 g) (37°C, shake culture). On the following day, the culture was diluted with 100 volumes of 2xT.Y.Amp. and further incubated. After 2 to 3 hours of incubation when the OD550 of the culture fluid had become 0.3 to 0.5, IPTG was added at a final concentration of 0.4 mM for inducing the production of recombinant human adiponectin (apM1) . About 3 to 5 hours after addition of IPTG, the culture fluid was centrifuged (5000 rpm, 20 min., 4°C) and the E. *coli* pellet thus obtained was freeze-preserved.

### 2) Preparation of an Inclusion Body from E. coli

The *E. coli* pellet was suspended in 50 mM Tris-HCl (pH 8.0) and treated with lysozyme at 37°C for 1 hour. Then, Triton X-100 (Katayama Kagaku) was added at a final concentration of 0.2%. This solution was sonicated (Branson Sonifier, output control 5, 30 sec.) and centrifuged (12000 rpm, 30 min, 4°C) and the pellet was recovered. This pellet was suspended in 25 ml of 0.2% Triton X-100-supplemented 50 mM Tris-HCl (pH 8.0) and the suspension was sonicated (under the same conditions as above).

The resulting solution was centrifuged and the pellet was washed by the same procedure as above. The pellet thus obtained was taken as the inclusion body.

### 3) Refolding of the Inclusion Body

The inclusion body was solubilized with a small quantity of 7 M guanidine HCl, 100 mM Tris-HCl (pH 8.0) and 1% 2ME. This solution was added dropwise into 200-fold volume of 2 M urea, 20 mM Tris-HCl (pH 8.0), diluted, and allowed to stand at 4°C for 3 nights.

### 4) Concentration of the Refolded Solution

The solution after the above refolding was centrifuged (9000 rpm, 30 min, 4°C) and the supernatant was concentrated to about 1/100 by ultrafiltration using an Amicon YM-10 membrane. This concentrate was dialyzed against 20 mM Tris-HCl (pH 8.0) and the dialysate was filtered through a 0.45 µm filter.

### 5) DEAE-5PW Anion-exchange HPLC

The sample obtained in the above step 4) was isolated and purified by anion-exchange high performance liquid chromatography (HPLC) with DEAE-5PW (Tosoh Corporation). As the starting buffer, 20 mM Tris-HCl (pH 7.2) was used, and elution was carried out with a NaCl gradient (0→1M NaCl/60 ml) under absorbance monitoring at 280 nm. The eluate was collected in 1 ml fractions and each fraction was analyzed by SDS-PAGE.

### 6) Results and Discussion

Because the recombinant human adiponectin (apM1) had been expressed as an inclusion body in E. *coli,* its purification was carried out by solubilization and refolding of the inclusion body. As a result, the recombinant human adiponectin was solubilized and separated on the anion-exchange column. The peak fractions (fraction Nos. 30-37) were analyzed by SDS-PAGE. As a result, an approximately 30 kD band was observed. In this analysis, a faint smear band was detected on the background, but as most of the protein was considered to be the recombinant human adiponectin (apM1), this approximately 30 kD band (recombinant human adiponectin) was used as the antigen in the subsequent immunization of rabbits and mice, and the active ingredient of the pulmonary disease therapeutic agents of the invention.

### (3) Preparation of Anti-Human Adiponectin Polyclonal and Monoclonal Antibodies

### 1) Preparation of the Polyclonal Antibody

The recombinant human adiponectin, 100 µg/body, was mixed with complete adjuvant in a 1:1 ratio and 5 rabbits were immunized with the mixture 8 times at 2-week intervals to obtain an anti-human adiponectin polyclonal antibody (Identification codes: OCT9101-OCT9105).

### 2) Preparation of the Monoclonal Antibody

The recombinant human adiponectin, 20 µg/body, was mixed with complete adjuvant in a 1:1 ratio and mice were immunized with the mixture 3 times at 2-week intervals. Then, the final immunization was carried out without the adjuvant 3 days before cell fusion. Cell fusion between the mouse spleen cell and myeloma cell was carried out by the PEG method and the hybridoma was selected in HAT medium.

Screening for a human adiponectin antibody-producing cell line was carried out by ELISA using the antigen (recombinant human adiponectin)-coated immunoplate, and the hybridoma was cloned by the limiting dilution method.

In the above manner, 11 anti-human adiponectin antibody producing hybridoma lines named KOC09101-KOC09111 were obtained. One hybridoma, among them, was deposited with the National Institute of Bioscience and Human Technology, the Ministry of International Trade and Industry, Japan (NIBH, Higashi 1-1-3, Tsukuba-shi, Ibaraki, Japan) as of Jun. 8, 1998 (original deposit date) (the identification code assigned by the depositor: KOC09108) and the demand for conversion to the deposit under Budapest Treaty was filed as of Oct. 7, 1998. The accession number of the final deposit is FERM BP-6542.

The hybridomas as single clones were respectively administered intraperitoneally to mice treated with pristane in advance and the ascites fluid was harvested (Identification codes: ANOC9101-9111).

### 3) Purification of Antibodies

The rabbit antiserum (polyclonal antibody) and mouse ascites fluid (monoclonal antibody) were respectively purified using a protein A column.

### 4) Expression of human adiponectin in Animal Cells

The cDNA of the adiponectin was excised with EcoRI and inserted into the EcoRI site of the expression vector pCIneo (Promega Corp.). The COS-1 cell (ATCC CRL1650) was transfected with the above pCIneo-human adiponectin using LlipofectAMINE (GIBCO BRL), and the culture supernatant and the cells were harvested after 72 hours.

### 5) Western Blotting of Human Adiponectin

First, the adipose tissue extract, COS-1 cells, COS-1 cell culture supernatant, healthy human plasma, and recombinant human adiponectin were subjected to 2 ME(+) SDS-PAGE and transferred to a nitrocellulose membrane.

This membrane was reacted with the anti-human adiponectin monoclonal antibody (ANOC9104) and, then, with the HRP-labeled antibody, and detection was carried out with ECL (Western blot detecting reagent, Amersham).

As a result, an approximately 35 kD band was detected for the adipose tissue extract, pCIneo-human adiponectin/COS-1 cell, and healthy human plasma, but was not observed for pCIneo/COS-1 cell or pCIneo/COS-1 cell culture supernatant.

With the culture supernatant of pCIneo-human adiponectin/COS-1 cells, a 35 kD band could be confirmed although it was too weak in intensity to be readily discernible.

### Reference Example 2

CHO cells that express N terminal His-Tag fusion mouse adiponectin (clone No. 5) were cultured on a large scale, and the resulting supernatant was collected. Using a Ni-NTA Resin, His-tagged adiponectin was purified.

### Reference Example 3

The full-length DNA, as shown in SEQ NO: 5, of mouse adiponectin was integrated into an expression vector and expressed in *E*. *coli,* providing a large amount of expressed mouse adiponectin. The supernatant of disrupted *E*. *coli* was applied to a DEAE-Sepharose open column, and elution was performed using a gradient from OM to 1M NaCl. The eluted fractions were then collected and subjected to fractionation with 30% saturated ammonium sulfate. The supernatant was applied to a Butyl-Toyopearl open column, and elution was performed with a gradient from 30% to 0% saturated ammonium sulfate. Finally, gel filtration was carried out to purify the mouse adiponectin.

### Example 1

Using model mice (CS7BL/6J) that had developed a lung disorder as a result of elastase treatment, adiponectin was examined for therapeutic effects on chronic obstructive pulmonary disease characterized by irreversible airflow limitation.

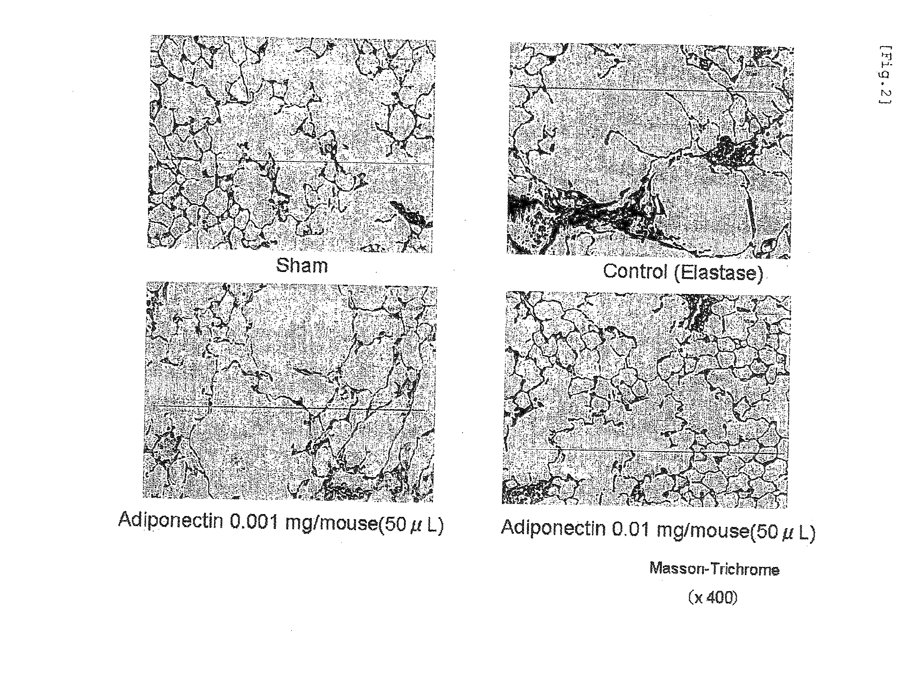

Once alveolar walls of the model mouse were destroyed by a one-time intratracheal administration of elastase, the alveolar wall destruction chronically continued thereafter, expanding emphysema lesions (control in Fig. 2). Such changes are indicated by mean linear intercept, which is a generally used evaluation index (Dunnill. M.S. Thorax (1962) 17, p320-328). The degree of alveolar wall destruction is evaluated by measuring the size of individual alveoli.

Thus, in the model mouse, the elastase treatment causes alveolar wall destruction, thereby resulting in expansion of the alveolar diameter, which is equivalent to emphysema lesions and pulmonary function impairment.

One-time intratracheal administration of elastase to mice resulted in the destruction of alveolar walls and a consequential breakdown of blood vessels, causing transient bleeding and neutrophil infiltration in the alveoli; however, this inflammation disappeared in three days. Therefore, in the experiment, it can be verified that the effect of adiponectin is not derived from elastase inhibitory action.

Female 6 or 7-week-old mice (C57BL/6L, Japan Charles River Co., Ltd.) were assigned to 4 groups (A to D, 6 mice per group) based on body weight using stratified randomization (SAS Institute Japan, R8.1), as shown in Table 1. Each mouse was subjected to pentobarbital anesthesia.

**Table 1**

| Group | Elastase treatment | Administration substance (Concentration) | Dose of adiponectin (mg/mouse) (50 µl) | Number of mice |
|---|---|---|---|---|
| A | Not performed | - | - | 6 |
| B | Performed | Normal saline solution | - | 6 |
| C | Performed | Adiponectin (0.02 mg/ml) | 0.001 | 6 |
| D | Performed | Adiponectin (0.2 mg/ml) | 0.01 | 6 |

Subsequently, using a sprayer (a product of Penn Century Inc.), normal saline solution (Otsuka normal saline, produced by Otsuka Pharmaceutical Factory Inc.) and human neutrophil elastase (produced by Elastin Products Co., Inc.) were intratracheally administered via the larynx to the mice in Group A and the mice in Groups B to D, respecitively, in a volume of 50 µl/mouse. The dosage of elastase was 20U/mouse. From three days after the intratracheal elastase administration, adiponectin was intratracheally administered once a day for 18 days to the mice in Group C at a dose of 0.001 mg/mouse and to the mice in Group D at a dose of 0.01 mg/mouse. The adiponectin used in the administration was obtained by dissolving in a solvent (50 mM Tris-HCl (pH 8.0) 0.5M NaCl) CHO cell-derived mouse adiponectin obtained in Reference Example 2 above. The adiponectin was diluted with a suitable amount of normal saline solution to a concentration of 0.2 mg/ml or 0.02 mg/ml in use. The solutions at each concentration were divided into daily dosage units, and freeze-preserved at -20°C until the date of administration. At the time of use, the frozen solution was thawed. In the elastase control group (Group B), normal saline solution was administered in place of adiponectin for the same period. Adiponectin or normal saline solution was intratracheally administered via the larynx to a mouse under isoflurane inhalation anesthesia at a volume of 50 µl/mouse using a sprayer. After 18 days of continuous intratracheal administration, each mouse was euthanized by bleeding from the abdominal aorta under isoflurane inhalation anesthesia. The lung was extirpated and subjected to perfusion fixation with a 10% neutral buffered formalin solution. The fixed-lung tissue was then subjected to paraffin embedding, thin sectioning, Masson Trichrome staining, and HE staining at a Bio Pathology Institute Co., Ltd. Pathology tissue was evaluated by measuring the mean linear intercept, which is an objective indicator of alveolar damage.

The mean linear intercept (Lm) was calculated using the formula: Lm = N x L / m, wherein N is the number of transversal lines, L is the length of a transversal line, and m is the total number of alveolar walls intersecting with the transversal lines.

### Statistical Analysis

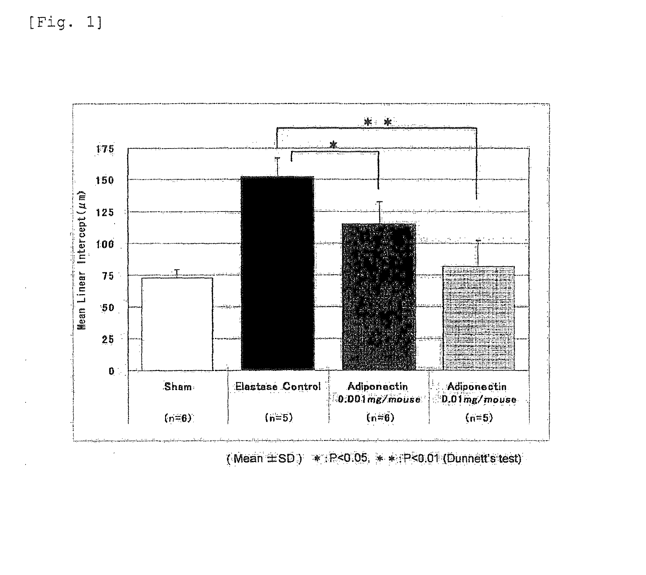

To examine the activities of the drugs, Dunnett's test was performed by comparing the elastase control group (Group B) with Groups C and D. Each test was conducted in accordance with the two-tailed test, and the significance level was set at 5%. The test was performed using SAS software (SAS Institute Japan, R8.1). The results are shown in Fig. 1.

The measured linear intercept was 152.5 ± 14.7 µm in Group B, while 115.2 ± 17.3 µm in Group C and 81.7 ± 20.4 µm in Group D. Significant inhibitory activities were demonstrated in accordance with the dosage of adiponectin (Mean ± S.D., P< 0.05). In Group A, the linear intercept was 73.0 ± 6.6 µm.

The results reveal that the continuous pulmonary administration of adiponectin has the dose-dependent activity of reducing the increase in mean linear intercept caused by elastase treatment.

Specifically, it was confirmed that the continuous administration of adiponectin suppressed alveolar airspace enlargement caused by progressing destructive changes of alveolar walls.

Fig. 2 shows tissue sections that indicate progressing destructive changes of alveolar walls. This reveals that the progressing alveolar wall destruction in groups receiving continuous administration of adiponectin (Groups C and D) is obviously more inhibited than that in the control group (Group B), and this inhibitory activity is greater in Group D.

Specifically, it was confirmed that the continuous administration of adiponectin suppressed the development of destructive changes of alveolar walls.

## Claims

1. An agent for inhibiting alveolar airspace enlargement, comprising adiponectin for use in the treatment of emphysema, chronic bronchitis, cystic fibrosis, bronchiectasis, tuberculosis, pneumoconiosis, or chronic obstructive pulmonary disease.

2. The agent according to Claim 1, wherein the adiponectin is contained in an amount of 0.01 to 70 wt. %.

3. An agent for inhibiting alveolar wall destruction, comprising adiponectin for use in the treatment of emphysema, chronic bronchitis, cystic fibrosis, bronchiectasis, tuberculosis, pneumoconiosis, or chronic obstructive pulmonary disease.

4. The agent according to Claim 3, wherein the adiponectin is contained in an amount of 0.01 to 70 wt. %.


SEQUENCE LISTING

<110>  OTSUKA PHARMACEUTICAL CO., LTD.
<120>  AGENT FOR TREATING PULMONARY DISEASE
<130>  P09-96
<150>  JP 2008-217721
<151>  2008-8-27
<160>  7
<170>  PatentIn version 3.1

<210>  1
<211>  244
<212>  PRT
<213>  homo sapiens
<400>  1
Met Leu Leu Leu Gly Ala Val Leu Leu Leu Leu Ala Leu Pro Gly His
1               5                   10                  15
Asp Gln Glu Thr Thr Thr Gln Gly Pro Gly Val Leu Leu Pro Leu Pro
            20                  25                  30
Lys Gly Ala Cys Thr Gly Trp Met Ala Gly Ile Pro Gly His Pro Gly
        35                  40                  45
His Asn Gly Ala Pro Gly Arg Asp Gly Arg Asp Gly Thr Pro Gly Glu
    50                  55                  60
Lys Gly Glu Lys Gly Asp Pro Gly Leu Ile Gly Pro Lys Gly Asp Ile
65                  70                  75                  80
Gly Glu Thr Gly Val Pro Gly Ala Glu Gly Pro Arg Gly Phe Pro Gly
                85                  90                  95
Ile Gln Gly Arg Lys Gly Glu Pro Gly Glu Gly Ala Tyr Val Tyr Arg
            100                 105                 110
Ser Ala Phe Ser Val Gly Leu Glu Thr Tyr Val Thr Ile Pro Asn Met
        115                 120                 125
Pro Ile Arg Phe Thr Lys Ile Phe Tyr Asn Gln Gln Asn His Tyr Asp
    130                 135                 140
Gly Ser Thr Gly Lys Phe His Cys Asn Ile Pro Gly Leu Tyr Tyr Phe
145                 150                 155                 160
Ala Tyr His Ile Thr Val Tyr Met Lys Asp Val Lys Val Ser Leu Phe
                165                 170                 175
Lys Lys Asp Lys Ala Met Leu Phe Thr Tyr Asp Gln Tyr Gln Glu Asn
            180                 185                 190
Asn Val Asp Gln Ala Ser Gly Ser Val Leu Leu His Leu Glu Val Gly
        195                 200                 205
Asp Gln Val Trp Leu Gln Val Tyr Gly Glu Gly Glu Arg Asn Gly Leu
    210                 215                 220
Tyr Ala Asp Asn Asp Asn Asp Ser Thr Phe Thr Gly Phe Leu Leu Tyr
225                 230                 235                 240
His Asp Thr Asn

<210>  2
<211>  735
<212>  DNA
<213>  homo sapiens
<220>
<221>  CDS
<222>  (1)...(735)
<400>  2
atgctgttgc tgggagctgt tctactgcta ttagctctgc ccgggcatga ccaggaaacc      60
acgactcaag ggcccggagt cctgcttccc ctgcccaagg gggcctgcac aggttggatg     120
gcgggcatcc cagggcatcc gggccataat ggggccccag gccgtgatgg cagagatggc     180
acccctggtg agaagggtga gaaaggagat ccaggtctta ttggtcctaa gggagacatc     240
ggtgaaaccg gagtacccgg ggctgaaggt ccccgaggct ttccgggaat ccaaggcagg     300
aaaggagaac ctggagaagg tgcctatgta taccgctcag cattcagtgt gggattggag     360
acttacgtta ctatccccaa catgcccatt cgctttacca agatcttcta caatcagcaa     420
aaccactatg atggctccac tggtaaattc cactgcaaca ttcctgggct gtactacttt     480

gcctaccaca tcacagtcta tatgaaggat gtgaaggtca gcctcttcaa gaaggacaag 540
gctatgctct tcacctatga tcagtaccag gaaaataatg tggaccaggc ctccggctct 600
gtgctcctgc atctggaggt gggcgaccaa gtctggctcc aggtgtatgg ggaaggagag 660
cgtaatggac tctatgctga taatgacaat gactccacct tcacaggctt tcttctctac 720
catgacacca actga 735

<210> 3
<211> 247
<212> PRT
<213> Mus musculus
<400> 3
Met Leu Leu Leu Gln Ala Leu Leu Phe Leu Leu Ile Leu Pro Ser His
1               5                   10                  15
Ala Glu Asp Asp Val Thr Thr Thr Glu Glu Leu Ala Pro Ala Leu Val
            20                  25                  30
Pro Pro Pro Lys Gly Thr Cys Ala Gly Trp Met Ala Gly Ile Pro Gly
        35                  40                  45
Ile Pro Gly Ile His Val Gly Thr Pro Gly Arg Asp Gly Arg Asp Gly
    50                  55                  60
Thr Pro Gly Glu Lys Gly Glu Lys Gly Asp Ala Gly Leu Leu Gly Pro
65                  70                  75                  80
Lys Gly Glu Thr Gly Asp Val Gly Met Thr Gly Ala Glu Gly Pro Arg
                85                  90                  95
Gly Phe Pro Gly Thr Pro Gly Arg Lys Gly Glu Pro Gly Glu Ala Ala
            100                 105                 110
Tyr Val Tyr Arg Ser Ala Phe Ser Val Gly Leu Glu Thr Arg Val Thr
        115                 120                 125
Val Pro Asn Val Pro Ile Arg Phe Thr Lys Ile Phe Tyr Asn Gln Gln
    130                 135                 140
Asn His Tyr Asp Gly Ser Thr Gly Lys Phe Tyr Cys Asn Ile Pro Gly
145                 150                 155                 160
Leu Tyr Tyr Phe Ser Tyr His Ile Thr Trp Met Lys Asp Val Lys Val
                165                 170                 175
Ser Leu Phe Lys Lys Asp Lys Ala Val Leu Phe Thr Tyr Asp Gln Tyr
            180                 185                 190
Gln Glu Lys Asn Val Asp Gln Ala Ser Gly Ser Val Leu Leu His Leu
        195                 200                 205
Glu Val Gly Asp Gln Val Trp Leu Gln Val Tyr Gly Asp Gly Asp His
    210                 215                 220
Asn Gly Leu Tyr Ala Asp Asn Val Asn Asp Ser Thr Phe Thr Gly Phe
225                 230                 235                 240
Leu Leu Tyr His Asp Thr Asn
                245

<210> 4
<211> 744
<212> DNA
<213> Mus musculus
<220>
<221> CDS
<222> (1)...(744)
<400> 4
atgctactgt tgcaagctct cctgttcctc ttaatcctgc ccagtcatgc cgaagatgac 60
gttactacaa ctgaagagct agctcctgct ttggtccctc cacccaaggg aacttgtgca 120
ggttggatgg caggcatccc aggacatcct ggccacaatg gcacaccagg ccgtgatggc 180
agagatggca ctcctggaga gaagggagag aaaggagatg caggtcttct tggtcctaag 240
ggtgagacag gagatgttgg aatgacagga gctgaagggc cacggggctt ccccggaacc 300
cctggcagga aaggagagcc tggagaagcc gcttatgtgt atcgctcagc gttcagtgtg 360
gggctggaga cccgcgtcac tgttcccaat gtacccattc gctttactaa gatcttctac 420
aaccaacaga atcattatga cggcagcact ggcaagttct actgcaacat tccgggactc 480
tactacttct cttaccacat cacggtgtac atgaaagatg tgaaggtgag cctcttcaag 540
aaggacaagg ccgttctctt cacctacgac cagtatcagg aaaagaatgt ggaccaggcc 600
tctggctctg tgctcctcca tctggaggtg ggagaccaag tctggctcca ggtgtatggg 660

gatggggacc acaatggact ctatgcagat aacgtcaacg actctacatt tactggcttt 720
cttctctacc atgataccaa ctga 744

<210> 5
<211> 1276
<212> DNA
<213> Mus musculus
<220>
<221> CDS
<222> (39)...(782)
<400> 5
ctctaaagat tgtcagtgga tctgacgaca ccaaaagggc tcaggatgct actgttgcaa 60
gctctcctgt tcctcttaat cctgcccagt catgccgaag atgacgttac tacaactgaa 120
gagctagctc ctgctttggt ccctccaccc aagggaactt gtgcaggttg gatggcaggc 180
atcccaggac atcctggcca caatggcaca ccaggccgtg atggcagaga tggcactcct 240
ggagagaagg gagagaaagg agatgcaggt cttcttggtc ctaagggtga gacaggagat 300
gttggaatga caggagctga agggccacgg ggcttccccg gaacccctgg caggaaagga 360
gagcctggag aagccgctta tatgtatcgc tcagcgttca gtgtggggct ggagacccgc 420
gtcactgttc ccaatgtacc cattcgcttt actaagatct tctacaacca acagaatcat 480
tatgacggca gcactggcaa gttctactgc aacattccgg gactctacta cttctcttac 540
cacatcacgg tgtacatgaa agatgtgaag gtgagcctct tcaagaagga caaggccgtt 600
ctcttcacct acgaccagta tcaggaaaag aatgtggacc aggcctctgg ctctgtgctc 660
ctccatctgg aggtgggaga ccaagtctgg ctccaggtgt atggggatgg ggaccacaat 720
ggactctatg cagataacgt caacgactct acatttactg gctttcttct ctaccatgat 780
accaactgac tgcaactacc catagcccat acaccaggag aatcatggaa cagtcgacac 840
actttcagct tagtttgaga gattgatttt attgcttagt ttgagagtcc tgagtattat 900
ccacacgtgt actcacttgt tcattaaacg actttataaa aaataatttg tgttcctagt 960
ccagaaaaaa aggcactccc tggtctccac gactcttaca tggtagcaat aacagaatga 1020
aaatcacatt tggtatgggg gcttcacaat attcgcatga ctgtctggaa gtagaccatg 1080
ctatttttct gctcactgta cacaaatatt gttcacataa accctataat gtaaatatga 1140
aatacagtga ttactcttct cacaggctga gtgtatgaat gtctaaagac ccataagtat 1200
taaagtggta gggataaatt ggaaaaaaaa aaaaaaaaaa agaaaaactt tagagcacac 1260
tggcggccgt tactag 1276

<210> 6
<211> 29
<212> DNA
<213> Artificial sequence
<220>
<223> Oligonucleotide as primer (forward) sequence for adiponectin
<400> 6
aacatatggg gcatgaccag gaaaccacg 29

<210> 7
<211> 29
<212> DNA
<213> Artificial sequence
<220>
<223> Oligonucleotide as primer (reverse) sequence for adiponectin
<400> 7
aaggatcctc agttggtgtc atggtagag 29